# EUROPÄISCHE PATENTSCHRIFT

(11) **EP 0 586 739 B1**
(45) Veröffentlichungstag und Bekanntmachung des Hinweises auf die Patenterteilung: **04.02.1998**
(21) Anmeldenummer: 92115567.7
(22) Anmeldetag: 11.09.1992
(51) Int. Cl.: A61N 1/365, A61N 1/39

(54) **Vorrichtung zum Behandeln von Tachyarrhythmien**
Means for treating tachyarrhythmia
Dispositif de traitement de tachyarhythmie

(43) Veröffentlichungstag der Anmeldung: 16.03.1994
(73) Patentinhaber: Pacesetter AB, 171 95 Solna (SE)
(72) Erfinder: Holmström, Nils, S-175 45 Järfälla (SE); Högnelid, Kurt, S-137 38 Västerhaninge (SE); Elmhammer, Agneta, S-115 24 Stockholm (SE); Hagel, Pia, S-191 50 Sollentuna (SE)
(74) Vertreter: Lettström, Richard Wilhelm

(56) Entgegenhaltungen:
- EP-A- 0 191 404
- US-A- 4 967 748
- US-A- 5 119 813

## Beschreibung

Die Erfinding betrifft eine Vorrichtung zum Behandeln von Tachyarrhythmien des Herzens mit Mitteln zum Detektieren solcher Tachyarrhythmien, an denen ausgangsseitig Mittel zur Auslösung der Tachyarrhythmiebehandlung angeschlossen sind.

Tachyarrhythmien sind Rhythmusstörungen des Herzens mit unnatürlich überhöhter Frequenz verursacht durch Veränderungen der Erregungsbildung oder Erregungsleitung im Herzen. Derartige Tachyarrhythmien führen zu einer reduzierten Kammerfüllung und Auswurfleistung des Herzens und können im schlimmsten Fall bei Vorliegen von Herzkammerflimmern zu einem Stillstand der Blutförderung durch das Herz führen.

Aus der EP-B-0 108 360 ist es bekannt, daß ein schneller Herzrhythmus, wenn er durch retrograde Überleitung verursacht ist, durch elektrische Stimulierungsimpulse eines Herzschrittmachers beendet werden kann, die zu bestimmten Zeitpunkten in bezug auf die Tachykardie-Herzschläge an das Herz abgegeben werden.

Aus der US-A-4 403 614 ist ein implantierbarer Kardiovertierer bekannt, bei dem durch Messen des Abstandes aufeinanderfolgender R-Wellen des Herzens die Herzfrequenz ermittelt wird. Dabei werden Rhythmusstörungen mit Frequenzen von weniger als 140 Schlägen je Minute als gutartig und Rhythmusstörungen mit Frequenzen zwischen 140 und 300 Schlägen je Minute als bösartig, weil in ein Herzkammerflimmern übergehend klassifiziert, während bei Frequenzen von mehr als 300 Schlägen je Minute ein Herzkammerflimmern detektiert wird. Wird eine als bösartig klassifizierte Tachyarrhythmie detektiert, so wird eine Kardiovertierung des Herzens mit einer zwischen der üblichen Stimulationsenergie von Herzschrittmacherimpulsen und der für Defibrillierungszwecke erforderlichen Energie liegenden Kardiovertierungsenergie ausgelöst. Bei der Detektion von Herzkammerflimmern erfolgt eine Defibrillation des Herzens.

Die unterschiedlichen Therapieverfahren: antitachykarde Stimulation, Kardioversion und Defibrillierung erfordern eine Erkennung der Rhythmusstörungen des Herzens. Dabei kann die Detektion von Tachyarrhythmien ausschließlich mit Hilfe von Herzschlagdetektoren und nachfolgender Auswertung herzschlagfrequenzbezogener Kriterien wie Frequenz, plötzlicher Frequenzanstieg und anhaltend hoher Frequenzwert problematisch sein. Insbesondere besteht die Gefahr, daß ein schneller Sinusrhythmus als Tachykardie mißinterpretiert wird.

Aus der US-A-3 942 536 ist ein implantierbarer Kardiovertierer bekannt, bei dem mit Hilfe eines Drucksensors am Ende eines Katheters der Druck im rechten Ventrikel erfaßt wird; sobald der gemessene Druck für eine vorgegebene Zeitdauer unter einen Grenzwert fällt, wird eine Kardioversion des Herzens ausgelöst. Bisher ist es aber noch nicht gelungen, für den obigen Zweck geeignete Drucksensoren herzustellen, die ausreichend empfindlich sind, deren Meßergebnisse durch Ablagerungen von fibrösem Gewebe nicht verfälscht werden und mit denen sich auch statische Drücke genau messen lassen.

In der US-A-3 805 795 ist vorgeschlagen, beispielsweise die mit einem Herzschlagdetektor erfaßte Herzschlagfrequenz als ein erstes Kriterium und die mit einem Bewegungssensor im Herzen erfaßte mechanische Herzaktivität als ein zweites Kriterium zur Detektion von Tachyarrhythmien heranzuziehen und eine Schockbehandlung des Herzens auszulösen, wenn beide Kriterien über eine vorgegebene Zeitdauer gleichzeitig erfüllt sind.

Aus der US-A-5 085 213 ist ein implantierbarer Defibrillator/Kardiovertierer bekannt, bei dem im Herzen gemessene Drücke und Frequenzkriterien zur Bestimmung von Herzrhythmusstörungen herangezogen werden. Hierbei ergibt sich ebenso wie bei der bereits genannten US-A-3 942 536 das Problem, daß geeignete Drucksensoren mit ausreichender Langzeitstabilität insbesondere für die Messung von statischen Drücken bisher nicht zur Verfügung stehen.

Aus der US-A-4 967 748 ist ein Verfahren zum Detektieren von Tachyarrhythmien bekannt, bei dem die Messung von im Blut vorhandenen Sauerstoff und das heranziehen einer ein vorgegebenes Mass überschreitenden Veränderung der gemessenen Sauerstoffanteile als Kriterium zur Detektion einer Tachyarrhythmie Verwendung findet.

Aus der EP-B-0 191 404 ist ein Herzschrittmachersystem mit Anpassung des Fluchtintervalls entsprechend dem Partialdruck eines Blutgases bekannt.

Der Erfindung liegt die Aufgabe zugrunde, eine sichere Detektion von schädlichen Tachyarrhythmien des Herzens zu ermöglichen.

Die Erfindung ist im Anspruch 1 definiert. Bevorzugte Ausführungsformen sind den Ansprüchen 2-3 zu entnehmen.

Bezüglich der eingangs genannten Vorrichtung wird die Aufgabe dadurch gelöst, daß die Mittel zum Detektieren von Tachyarrhythmien einen Sensor zur Bestimmung des Partialdruckes von im Blut gelösten Gasen aufweisen und daß dem Sensor eine Auswerteeinrichtung zur Erkennung von schnellen und/oder ein vorgegebenes Maß überschreitenden Veränderungen des gemessenen Partialdrucks nachgeordnet ist. Bei der erfindungsgemäßen Vorrichtung zum Behandeln von Tachyarrhythmien kann es sich um einen Antitachykardie-Herzschrittmacher, Konvertierer oder Defibrillator handeln.

Wenn eine Tachyarrhythmie startet, führt dies zu einem sehr schnellen Druckabfall im Herzen, so daß sich der Partialdruck der in dem Blut gelösten Gase sehr schnell ändert. Mit der Erfassung des Partialdrucks gelingt es also in vorteilhafter Weise Tachyarrhythmien nicht indirekt über die Herzschlagfrequenz sondern vielmehr über ihre hämodynamischen Auswirkungen zu diagnostizieren. Im unterschied zu Drucksensoren sind Sensoren zur Bestimmung von Partialdrücken einfach als Kathetersensoren zu realisieren und weisen eine hohe Langzeitstabilität auf. Derartige Sensoren zur Messung der Sauerstoffkonzentration sind beispielsweise in den Schutzrechtsveröffentlichungen US-A-4 779 618, US-A-4 853 091, EP-A-0 455 072 und WO 91/17433 beschrieben.

Aus der US-A-4 779 618 ist ferner die Verwendung eines solchen Sauerstoffsensors zur Frequenzsteuerung eines Herzschrittmachers bekannt, jedoch wird dabei die durch die sogenannte Sauerstoffbindungskurve des Blutes definierte Abhängigkeit des Sauerstoffgehalts des Blutes vom Partialdruck des Sauerstoffs ausgenutzt. Da jedoch nur sehr wenig Sauerstoff physikalisch im Blut gelöst ist, entspricht der Sauerstoffgehalt des Blutes weitgehend dem chemisch an Hämoglobin gebundenen Sauerstoff, also der Sauerstoffsättigung des Blutes, die ein metabolischer Parameter ist und als belastungsabhängige Regelgröße in dem bekannten frequenzgesteuerten Herzschrittmacher genutzt wird. Im Unterschied hierzu wird entsprechend einer Weiterbildung der erfindungsgemäßen Vorrichtung der Partialdruck des Sauerstoffs im Blut gemessen und ein schnelles Absinken des gemessenen Partialdrucks und/oder ein Absinken des gemessenen Partialdrucks unter einen vorgegebenen Mindestwert als Kriterium zur Detektion einer Tachyarrhythmie herangezogen; dementsprechend ist gemäß einer Weiterbildung der erfindungsgemäßen Vorrichtung der Sensor ein Sauerstoffsensor und die Auswerteeinrichtung zur Detektion eines plötzlichen und/oder einen vorgegebenen Mindestwert unterschreitenden Absinkens des gemessenen senen Sauerstoffpartialdrucks ausgebildet. Dabei wird im Unterschied zu dem oben genannten bekannten Herzschrittmacher der Umstand ausgenutzt, daß bei einem plötzlichen Druckabfall im Herzen aufgrund tachykarder Rhythmusstörungen der Partialdruck des Sauerstoffs im Blut ebenso schnell absinkt, und zur Detektion einer Tachyarrhythmie herangezogen. Wenn dabei der Sauerstoffpartialdruck den vorgegebenen Mindestwert unterschreitet, weist dies darauf hin, daß die Blutförderung und damit die Aufladung des Bluts mit Sauerstoff praktisch zum Erliegen gekommen ist, woraus direkt das Vorliegen von Herzkammerflimmern detektiert werden kann.

Bei einer vorteilhaften Weiterbildung der erfindungsgemäßen Vorrichtung wird als weiteres Kriterium zur Detektion einer Tachyarrhythmie die Herzschlagfrequenz erfaßt und ausgewertet und das gleichzeitige Vorliegen beider Kriterien zur Detektion einer Tachyarrhythmie herangezogen. Im diesen Zusammenhang ist bei der erfindungsgemäßen Vorrichtung ein Herzschlagdetektor vorgesehen, dem eine weitere Auswerteeinrichtung zur Bestimmung der Herzschlagfrequenz nachgeordnet ist, wobei die beiden Auswerteeinrichtungen ausgangsseitig über ein UND-Glied mit den Mitteln zur Auslösung der Tachyarrhythmiebehandlung verbunden sind. Hierdurch gelingt es, Tachyarrhythmien sowohl durch ihre Frequenz also auch durch ihre hämodynamischen Auswirkungen zu detektieren. Die Auswertung der Herzschlagfrequenz erfolgt dabei in bekannter Weise über das EKG des Herzens und kann Kriterien wie das Vorliegen einer überhöhten Frequenz, eines plötzlichen Frequenzanstiegs oder einer anhaltend überhöhten Frequenz umfassen.

Bei einer vorteilhaften Weiterentwicklung der erfindungsgemäßen Vorrichtung wird mit einem Aktivitätssensor die körperliche Aktivität des Patienten erfaßt und eine niedrige oder unveränderte gemessene Aktivität bei gleichzeitiger Veränderung des Partialdrucks als Kriterium zur Detektion einer Tachyarrhytmie herangezogen. Hierdurch wird die Detektionssicherheit weiter erhöht. Eine Steigerung der körperlichen Aktivität führt nämlich zu einem erhöhten Sauerstoffbedarf und damit zu einem Absinken des Sauerstoffpartialdrucks. Wenn daher ein Absinken des Sauerstoffpartialdrucks gemessen wird, ohne daß eine Steigerung der körperlichen Aktivität vorliegt oder wenn das Aktivitätsniveau gleichzeitig sehr gering ist, so liegt eine die Blutförderung und damit die Sauerstoffaufladung des Blut beinträchtigende Fehlfunktion des Herzens vor.

Zur Erläuterung der Erfindung wird im folgenden auf die Figuren der Zeichnung Bezug genommen; im einzelnen zeigen
- Figur 1: das Verhalten des Sauerstoffpartialdrucks pO₂ im rechten Atrium und des arteriellen Drucks aP bei überhöhten stimulierten Herzschlagfrequenzen und
- Figur 2: ein Ausführungsbeispiel für die erfindungsgemäße Vorrichtung zur Tachyarrhythmiebehandlung in Form eines Antitachykardie-Herzschrittmachers.

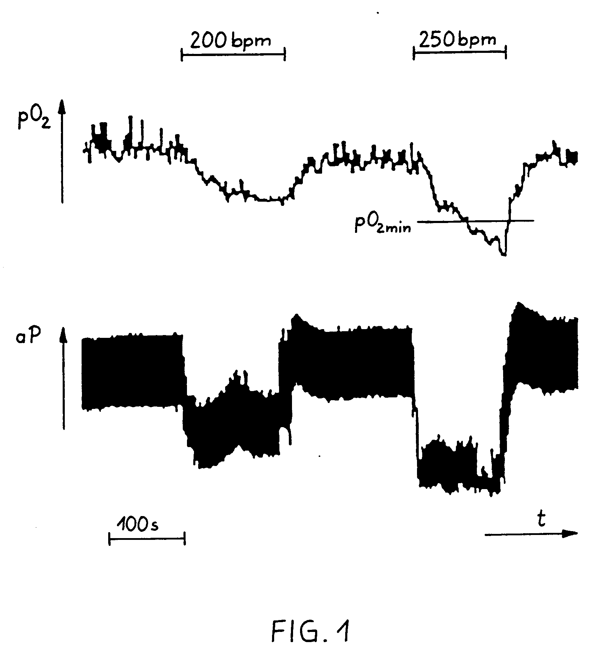

Figur 1 zeigt im oberen Teil der Abbildung den im rechten Atrium des Herzens gemessenen Verlauf des Sauerstoffpartialdrucks pO₂ und im unteren Teil der Abbildung den zugehörigen Verlauf des arteriellen Drucks aP, wobei die Schlagfrequenz des spontan schlagenden Herzens durch Stimulation mittels eines Herzschrittmachers in einem ersten Intervall auf 200 Schläge je Minute und in einem zweiten Intervall auf 250 Schläge je Minute angehoben wird. Bei diesen hohen Frequenzwerten verschlechtert sich die Kammerfüllung und Auswurfleistung des Herzens dermaßen, daß dies nicht oder nur unzureichend von der körpereigenen Kreislaufregelung kompensiert werden kann. Wie Figur 1 zeigt, führt die Stimulation des Herzens mit Frequenzen von 200 bzw. 250 Schlägen je Minute zu einem plötzlichen arteriellen Druckabfall, dem eine ebenso rasche Reaktion des Sauerstoffpartialdrucks pO₂ entspricht.

Der Sauerstoffpartialdruck pO₂ ist also eine sehr schnelle und sensitive Grösse zur Erfassung von Tachyarrhythmien. Im Falle der Stimulation mit 200 Schlägen je Minute stabilisiert sich der Sauerstoffpartialdruck pO₂ nach einiger Zeit auf einem niedrigeren Niveau, was auf eine verminderte Durchblutung und damit verminderte Sauerstoffsättigung des Blutes zurückzuführen ist. Bei der Stimulation des Herzens mit 250 Schläge je Minute sinkt dagegen der Sauerstoffpartialdruck pO₂ kontinuierlich ab, weil die Blutförderung und damit die Aufladung des Bluts mit Sauerstoff praktisch zum Stillstand gekommen ist. Dieselbe Situation würde also auch bei einem Herzkammerflimmern eintreten, was entsprechend dem erfindungsgemäßen Verfahren dadurch detektiert wird, daß der Sauerstoffpartialdruck pO₂ einen Mindestwert pO₂ₘᵢₙ unterschreitet.

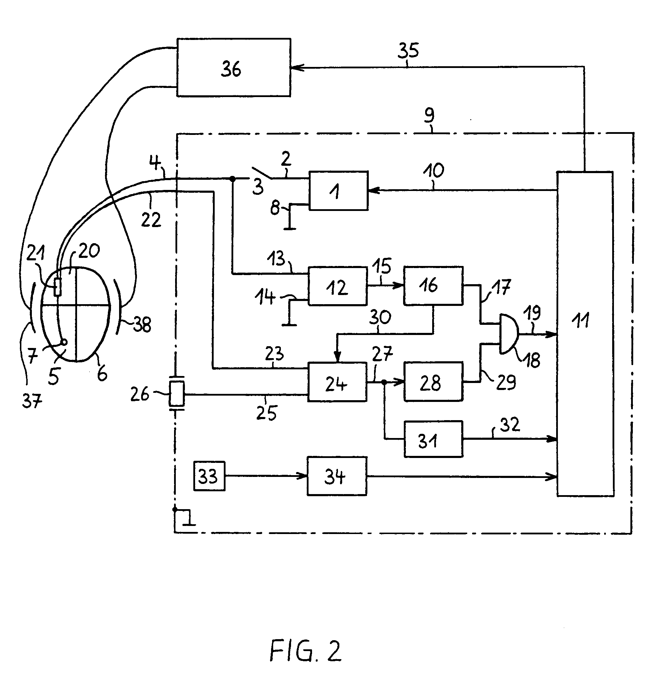

Figur 2 zeigt das Blockschaltbild eines Antitachykardie-Herzschrittmachers bei dem der Partialdruck des Sauerstoffs pO₂ im Blut zur Detektion von Tachyarrhythmien herangezogen wird. Der Herzschrittmacher enthält einen Stimulationsimpulsgenerator 1, der an seinem Ausgangsanschluß 2 über einen steuerbaren Schalter 3 und eine Elektrodenleitung 4 mit einer im Ventrikel 5 des Herzens 6 angeordneten Stimulationselektrode 7 beispielsweise aus aktivierten Glaskohlenstoff verbunden ist. Der zweite Ausgangsanschluß 8 des Stimulationsimpulsgenerators 1 ist mit einem Gehäuse 9 des Herzschrittmachers verbunden, das als Gegenelektrode zur Stimulationselektrode 7 dient. Der Stimulationsimpulsgenerator 1 ist über eine Steuerleitung 10 mit einer Herzschrittmachersteuerung 11 verbunden, die über die Steuerleitung 10 die Abgabe von Stimulationsimpulsen durch den Stimulationsimpulsgenerator 1 veranlaßt. Ein Herzschlagdetektor 12 ist zur Detektion von stimulierten oder natürlichen elektrischen Herzaktivitäten mit einem ersten Eingangsanschluß 13 an der Stimulationselektrode 7 und mit einem zweiten Eingangsanschluß 14 an dem Herzschrittmachergehäuse 9 angeschlossen. An dem Ausgang 15 des Herzschlagdetektors 12 ist eine Auswerteeinrichtung 16 angeschlossen, die die detektierten Herzschläge bezüglich ihrer Frequenz auswertet. Die Auswerteeinrichtung 16 ist über eine Ausgangsleitung 17 mit einem ersten Eingang eines UND-Glieds 18 verbunden, das mit seinem Ausgang 19 an der Herzschrittmachersteuerung 11 angeschlossen ist.

Im rechten Atrium 20 des Herzens 6 ist eine Sauerstoffmeßelektrode 21 vorzugsweise aus glattem, elektrokatalytisch inaktivem Glaskohlenstoff angeordnet, die über eine weitere Elektrodenleitung 22 mit einem ersten Eingang 23 einer Sensorelektronik 24 verbunden ist, an deren zweiten Eingang 25 eine Silber-Silberchlorid-Bezugselektrode 26 angeschlossen ist. Die Sauerstoffmeßelektrode 21 ist vorzugsweise als Ringelektrode ausgebildet, die von der als Spitzenelektrode ausgebildeten Stimulationselektrode 7 in proximaler Richtung beabstandet an einem beiden Elektroden 7 und 21 gemeinsamen, hier jedoch nicht gezeigten Katheter angeordnet ist.

Die Sauerstoffmeßelektrode 21 kann alternativ zu dem gezeigten Ausführungsbeispiel zusätzlich zur Erfassung der Herzschläge herangezogen werden, indem der Eingangsanschluß 13 des Herzschlagdetektors 12 anstelle mit der Stimulationselektrode 7 mit der Sauerstoffmeßelektrode 21 verbunden ist oder indem der Eingangsanschluß 14 des Herzschlagdetektors 12 anstelle mit dem Herzschrittmachergehäuse 9 mit der Sauerstoffmeßelektrode 21 verbunden ist.

Die Bezugselektrode 26 ist vorzugsweise im Bereich des Herzschrittmachergehäuses 9 gegenüber diesem isoliert angeordnet, kann aber auch an einem Katheter angeordnet sein. Der von den Elektroden 21 und 26 und der Sensorelektronik 24 gebildete Sauerstoffsensor funktioniert in der Weise, daß während vorgegebene Zeiten außerhalb der Stimulations des Herzens 6 zwischen den Elektroden 21 und 26 eine oder mehrere Meßspannungen erzeugt werden und der daraus resultierende Stromfluß in der Sauerstoffmeßelektrode 21 gemessen und aufintegriert wird, wobei an dem Ausgang 27 der Sensorelektronik 24 ein dem Sauerstoffpartialdruck pO₂ entsprechender Wert erhalten wird. Im übrigen wird auf die vorstehend bereits genannten Schutzrechtsveröffentlichungen US-A-4 779 618, US-A-4 853 091, EP-A-0 455 072 und WO 91/17433 verwiesen.

An dem Ausgang 27 der Sensorelektronik 24 ist eine Auswerteeinrichtung 28 angeschlossen, in der ein plötzliches Absinken des gemessenen Wertes für den Sauerstoffpartialdruck pO₂ detektiert wird. Die Auswerteeinrichtung 28 ist über eine Ausgangsleitung 29 mit einem zweiten Eingang des UND-Gliedes 18 verbunden. Um den Stromverbrauch durch die Messung des Sauerstoffpartialdrucks pO₂ zu verringern, ist die Sensorelektronik 24 über eine Steuerleitung 30 mit der dem Herzschlagdetektor 12 nachgeordneten Auswerteeinrichtung 16 verbunden und wird durch diese erst dann aktiviert, wenn die gemessene Herzschlagfrequenz einen vorgegebenen Wert von beispielsweise 110 Schlägen je Minute überschreitet.

Eine an dem Ausgang 27 der Sensorelektronik 24 angeschlossene weitere Auswerteeinrichtung 31 gibt über ihre Ausgangsleitung 32 an die Herzschrittmachersteuerung 11 ein Signal ab, wenn der gemessene Sauerstoffpartialdruck pO₂ den vorgegebenen Mindestwert pO₂ₘᵢₙ unterschreitet. Schließlich ist in dem Gehäuse 9 ein Aktivitätssensor 33 wie zB. ein Beschleunigungssensor angeordnet, der über eine eigene Sensorelektronik 34 an der Herzschrittmachersteuerung 11 angeschlossen ist.

Der Herzschlagdetektor 12 detektiert die Herzschlagfrequenz bzw. die Frequenz der elektrischen Aktivität des Herzens 6. Die Auswerteeinrichtung 16 wertet die gemessene Herzschlagfrequenz in der Weise aus, daß beim Überschreiten der vorgegebenenen Grenzfrequenz von etwa 110 Schlägen je Minute die Sensorelektronik 24 aktiviert wird. Bei einem weiteren, plötzlichen Ansteigen der Herzschlagfrequenz und Überschreiten eines weiteren vorgegebenen Grenzwertes während einer vorgegebenen Dauer erzeugt die Auswerteeinrichtung 16 auf ihrer Ausgangsleitung 17 ein Ausgangssignal als Kriterium für das Vorliegen einer Tachyarrhythmie. Die der Sensorelektronik 24 nachgeordnete Auswerteeinrichtung 28 erzeugt auf ihrer Ausgangsleitung 29 ein Ausgangssignal, wenn der gemessene Wert für den Sauerstoffpartialdruck pO₂ plötzlich einen vorgegebenen Grenzwert unterschreitet bzw. innerhalb einer vorgebenen Zeit um einen vorgebenen Betrag absinkt. Das Ausgangssignal auf der Ausgangsleitung 29 bildet ein zweites Kriterium für das Vorliegen einer Tachyarrhythmie, welches mit dem ersten Kriterium auf der Ausgangsleitung 17 durch das UND-Glied 18 in der Weise verknüpft wird, daß der Herzschrittmachersteuerung 11 über den Ausgang 19 des UND-Glieds 18 dann das Vorliegen einer Tachyarrhythmie, hier zB. einer Tachykardie mitgeteilt wird, wenn beide Kriterien gleichzeitig erfüllt sind. In diesem Fall veranlaßt die Herzschrittmachersteuerung 11 über die Steuerleitung 10 den Stimulationsimpulsgenerator 1 zur Abgabe einer programmgemäßen Folge von Stimulationsimpulsen zum Beenden der Tachykardie. Beispiele für entsprechende Stimulationsimpulse zur Beendigung einer Tachyarrhythmie sind aus der eingangs bereits genannten EP-B-0 108 360 bekannt.

Wenn der gemessene Sauerstoffpartialdruck pO₂ unter den vorgegebenen Mindestwert pO₂ₘᵢₙ fällt, wird dies durch die weitere Auswerteeinrichtung 31 als die Folge von Herzkammerflimmern detektiert und der Herzschrittmachersteuerung 11 mitgeteilt. Diese gibt daraufhin über eine Steuerleitung 35 oder eine hierzu alternative drahtlose Signalübertragungsstrecke ein Steuersignal an einen Defibrillator 36 ab, der einen Defibrillierungsimpuls über Defibrillierungselektroden 37 und 38 an das Herz 6 abgibt.

Eine weitergehende Klassifizierung unterschiedlicher Tachyarrhythmien kann beispielsweise in der Weise erfolgen, wie sie aus der eingangs genannten US-A-4 403 614 oder US-A-5 085 213 bekannt ist, wobei als zusätzliches bzw. zur Druckmessung im Herzen alternatives Kriterium zur Detektion der Tachyarrhythmie das plötzliche Absinken des Sauerstoffpartialdruckes im Blut herangezogen wird.

Die Sicherheit bei der Detektion von Tachyarrhythmien wird noch dadurch erhöht, daß bei einem plötzlichen bzw. erheblichen Absinken des Sauerstoffpartialdrucks pO₂ in der Herzschrittmachersteuerung 11 die von dem Aktivitätssensor 33 erfaßte körperliche Aktivität des Patienten zur Kontrolle herangezogen wird, ob das Absinken des Sauerstoffpartialdrucks pO₂ nicht auf einer erhöhten körperlichen Aktivität beruht. Wird dagegen ein Absinken des Sauerstoffpartialdrucks bei unveränderter oder niedriger körperlicher Aktivität des Patienten gemessen, so ist dies ein Kriterium für das Vorliegen einer Tachykardie.

### Bezugszeichenliste

- 1: Stimulationsimpulsgenerator
- 2: Ausgangsanschluß von 1
- 3: steuerbarer Schalter
- 4: Elektrodenleitung
- 5: Ventrikel von 6
- 6: Herz
- 7: Stimulationselektrode
- 8: zweiter Ausgangsanschluß von 1
- 9: Herzschrittmachergehäuse
- 10: Steuerleitung
- 11: Herzschrittmachersteuerung
- 12: Herzschlagdetektor
- 13: erster Eingangsanschluß von 12
- 14: zweiter Eingangsanschluß von 12
- 15: Ausgang von 12
- 16: Auswerteeinrichtung
- 17: Ausgangsleitung von 16
- 18: UND-Glied
- 19: Ausgang von 18
- 20: Atrium von 6
- 21: Sauerstoffmeßelektrode
- 22: weitere Elektrodenleitung
- 23: erster Eingang von 24
- 24: Sensorelektronik
- 25: zweiter Eingang von 24
- 26: Silberchlorid-Bezugselektrode
- 27: Ausgang von 24
- 28: Auswerteeinrichtung
- 29: Ausgangsleitung von 28
- 30: Steuerleitung
- 31: weitere Auswerteeinrichtung
- 32: Ausgangsleitung von 31
- 33: Aktivitätssensor
- 34: Sensorelektronik
- 35: Steuerleitung
- 36: Defibrillator
- 37,38: Defibrillierungselektroden
- aP: arterieller Druck
- pO₂: Partialdruck des Sauerstoffs im Blut
- pO₂ₘᵢₙ: vorgegebener Mindestwert für pO₂

## Patentansprüche

1. Vorrichtung zum Behandeln von Tachyarrhythmien des Herzens (6) mit Mitteln (21-26) zum Detektieren von Tachyarrhythmien, an denen ausgangsseitig Mittel (1, 11, 36) zur Auslösung der Tachyarrhythmiebehandlung angeschlossen sind, wobei die Mittel zum Detektieren von Tachyarrhythmien einen Sensor (21-26) zur Bestimmung des Partialdrucks von im Blut gelösten Gasen aufweisen und dem Sensor (21-26) eine Auswerteeinrichtung (28, 31) zur Erkennung von schnellen und/oder ein vorgegebenes Maß überschreitenden Veränderungen des gemessenen Partialdrucks nachgeordnet ist.

2. Vorrichtung nach Anspruch 1, **dadurch gekennzeichnet**, daß der Sensor (21-26) ein Sauerstoffsensor ist und die Auswerteeinrichtung (28) zur Detektion eines plötzlichen und/oder einen vorgegebenen Mindestwert (pO₂ₘᵢₙ) unterschreitenden Absinkens des gemessenen Sauerstoffpartialdrucks (pO₂) ausgebildet ist.

3. Vorrichtung nach Anspruch 1 oder 2, **dadurch gekennzeichnet**, daß ein Herzschlagdetektor (12) vorgesehen ist, dem eine weitere Auswerteeinrichtung (16) zur Bestimmung der Herzschlagfrequenz nachgeordnet ist, und daß die beiden Auswerteeinrichtungen (16 und 28) ausgangsseitig über ein UND-Glied (18) mit den Mitteln (1, 11) zur Auslösung der Tachyarrhythmiebehandlung verbunden sind.

## Claims

1. Apparatus for the treatment of tachyarrhythmia of the heart (6), having means (21-26) for detecting tachyarrhythmia, connected to which means (21-26), on the output side, there are means (1, 11, 36) for initiating the tachyarrhythmia treatment, wherein the means for detecting tachyarrhythmia have a sensor (21-26) for determining the partial pressure of gases dissolved in the blood and arranged downstream of the sensor (21-26) there is an evaluating devise (28, 31) for identifying changes in the measured partial pressure that are rapid and/or exceed a given measure.

2. Apparatus according to claim 1, characterised in that the sensor (21-26) is an oxygen sensor and the evaluating device (28) is formed for the purpose of detecting a drop in the measured partial oxygen pressure (pO₂) that is sudden and/or falls below a given minimum value (pO₂ₘᵢₙ).

3. Apparatus according to claim 1 or 2, characterised in that a heart beat detector (12) is provided, arranged downstream of which there is a further evaluating device (16) for the purpose of determining the heart beat frequency, and in that on the output side the two evaluating devices (16 and 28) are connected, by way of an AND element (18), to the means (1, 11) for initiating the tachyarrhythmia treatment.

## Revendications

1. Dispositif pour traiter des tachyarythmies du coeur (6), comportant des moyens (21 à 26) destinés à détecter des tachyarythmies, auxquels sont reliés, du côté de la sortie, des moyens (1, 11, 36) destinés à déclencher le traitement de tachyarythmie, les moyens destinés à détecter des tachyarythmies comportant un capteur (21 à 26) servant à déterminer la pression partielle de gaz dissous dans le sang et un dispositif (28, 31) d'exploitation, destiné à reconnaître des modifications rapides de la pression partielle mesurée et/ou des modifications de ladite pression dépassant une valeur prescrite, disposé en aval du capteur (21 à 26).

2. Dispositif suivant la revendication 1, caractérisé en ce que le capteur (21 à 26) est un capteur d'oxygène et le dispositif (28) d'exploitation est constitué de manière à détecter une chute soudaine et/ou un passage endessous d'une valeur (pO₂ₘᵢₙ) minimale prescrite de la pression partielle (pO₂) d'oxygène.

3. Dispositif suivant la revendication 1 ou 2, caractérisé en ce qu'il est prévu un capteur (12) de battement cardiaque, en aval duquel est disposé un dispositif (16) d'exploitation supplémentaire pour la détermination du pouls et en ce que les deux dispositifs (16 et 28) d'exploitation sont reliés, du côté de la sortie, par un élément (18) ET aux moyens (1, 11) destinés à déclencher le traitement de la tachyarythmie.
